(19) Europäisches Patentamt / European Patent Office / Office européen des brevets

(11) **EP 1 021 208 B1**

(12) **EUROPÄISCHE PATENTSCHRIFT**

(45) Veröffentlichungstag und Bekanntmachung des Hinweises auf die Patenterteilung:
**27.05.2009 Patentblatt 2009/22**

(21) Anmeldenummer: **99938380.5**

(22) Anmeldetag: **30.07.1999**

(51) Int Cl.:
*A61L 2/16* *(2006.01)*    *A61L 31/16* *(2006.01)*
*A61B 19/08* *(2006.01)*

(86) Internationale Anmeldenummer:
**PCT/EP1999/005455**

(87) Internationale Veröffentlichungsnummer:
**WO 2000/007633 (17.02.2000 Gazette 2000/07)**

(54) **MEDIZINISCHES GERÄT**

MEDICAL APPARATUS

APPAREIL MEDICAL

(84) Benannte Vertragsstaaten:
**CH DE FR GB LI**

(30) Priorität: **07.08.1998 CH 164598**

(43) Veröffentlichungstag der Anmeldung:
**26.07.2000 Patentblatt 2000/30**

(73) Patentinhaber: **Leica Microsystems (Schweiz) AG
9435 Heerbrugg (CH)**

(72) Erfinder: **PENSEL, Jürgen
CH-9450 Altstätten (CH)**

(74) Vertreter: **Rosenich, Paul
Patentbüro Paul Rosenich AG
BGZ
9497 Triesenberg (LI)**

(56) Entgegenhaltungen:
**EP-A2- 0 405 284        EP-A2- 0 472 413
WO-A1-92/11043        WO-A1-95/19583
WO-A2-97/29778        US-A- 5 681 575**

**Beschreibung**

Die Erfindung betrifft ein Operationsmikrop und/oder dessen Stativ mit einer, insbesondere der Umgebung zugewandten, Oberflächen. Ausdrücklich nicht umfaßt sind durch die Erfindung medizinische, nicht optische Instrumente für invarsive Eingriffe.

Seit jeher ist es Absicht, bei Operationen und/ oder im Bereich der medizinischen Forschung steril zu arbeiten. Das heißt, die zu operierenden Patienten und/ oder die der Forschung unterliegenden Objekte sollen möglichst nicht durch Keime (Bakterien, Viren, Pilze oder dergleichen) kontaminiert werden. Darüber hinaus werden durch die komplette Abhaltung bzw. Entfernung von jeglichen Verunreinigungen, insbesondere von Eiweiß-Substanzen allergische Reaktionen beim Patienten vermieden, der unabsichtlich und ungeschützt mit den medizinischen Geräten in Berührung kommt. Dies wird in der Regel dadurch erreicht, daß die Oberflächen der medizinischen Geräte peinlichst genau gesäubert bzw. sterilisiert werden. Jene Oberflächen, die schlecht sterilisierbar sind, werden abgedeckt mit sterilen Tüchern oder Folien. Beim Operationsmikroskop muß typischerweise ein "Drape", die Oberfläche des Mikroskops und Stativs mindestens im Sterilbereich um den Patienten vollständig umhüllen bzw. abschirmen. Ein solches Drape ist beispielsweise in der DE-A1-44 13 920 beschrieben. Das Drape ist in der Regel ein Wegwerfartikel, so daß seine Anwendung auch mit Kosten und mit einer direkten Umweltbelastung verbunden ist. Die Entsorgung von Wegwerfartikeln aus einem Operationsraum ist teuer.

Abgesehen davon, daß das Abdecken und Sterilisieren sowie die Entsorgung des kontaminierten Materials ein zeitaufwendiger und kostspieliger Vorgang ist, ist die Gefahr verbleibender unsteriler Lücken immer gegeben. Zudem reduzieren Drapes in der Regel die optischen Qualitäten von Mikroskopen, da sie zwangsläufig auch die Optik mit einschließen. Die dafür verwendeten Abdeckgläser können nur schlecht dampfsterilisiert werden, da verbleibende Wasserflecken die Optik zusätzlich stören. Außerdem engen die Abdeckungen die Bewegungsfreiheit und die Sicht im Sterilbereich ein.

Olympus hat ein Mikroskop CHK2 und ein Mikroskop CHL2 auf den Markt gebracht, deren optischen Teile während der Fertigung ständig von einem dünnen, dampfförmigen Anti-Pilz-Gas umströmt werden, das das Wachstum von Pilzen über einen Zeitraum von drei Jahren wirksam verhindert. Ein dicht versiegelter Binokulartubus wird noch weitergehender gegen Pilzbefall geschützt, weil weder Verunreinigungen noch Feuchtigkeit in das Mikroskop eindringen können. Bei dieser Antipilzausrüstung ging es um einen Schutz der optischen Teile, denn Pilzbefall führte oft zu Schäden an den optischen Teilen eines Mikroskops, besonders in tropischen Regionen. Insofern verbessert die durch Olympus vorgesehene Anti-Pilz-Beschichtung der Optik jedoch nicht die Sterilität des Mikroskops. Es wird lediglich die Lebensdauer der Optik bzw. deren optische Eigenschaften verlängert. Der Schutz von Patienten war bei dieser Art von Antipilzausrüstung nicht im Blickfeld der Hersteller.

WO 92/11043 beschreibt eine antimikrobielle Oberfläche medizinische produckte. Die Oberfläche kann mit einer Vielzahl von Erhebungen bzw. Vertielungen verschen werden. US 5 681 575 offenbart eine undere antimikrobielle Oberfläche für medizinische Produkte.

Im Zuge von Vereinfachungsbestrebungen bzw. Bestrebungen zur Erhöhung der Sterilität stellt sich der Erfindung die Aufgabe, Maßnahmen zu finden, die die Reinigung und/oder die Sterilität von medizinischen Geräten erleichtern bzw. verbessern.

Gelöst wird diese Aufgabe durch die Anwendung der Merkmale des Anspruches 1.

Durch eine spezielle Ausbildung und/oder Beschichtung der Oberfläche bzw. des Materials der medizinischen Geräte, so daß diese bzw. dieses keim/ schmutzabweisend oder keimtötend wirkt, kann auf Abdeckungen (Drapes) verzichtet werden bzw. wird die Sicherheit sogar unter sterilen Bedingungen noch erhöht.

Keimabweisende Beschichtungen sind zwar im Bereich von medizinischen Implantaten, wie künstlichen Herzklappen, Prothesen o.dgl. schon bekannt, jedoch hat offensichtlich noch niemand daran gedacht, diese an sich bekannten Maßnahmen auch an medizinischen Geräten, insbesondere Mikroskopen und deren Stativen anzuwenden, wo sich erst die oben geforderten bzw. beschriebenen erfindungsgemäßen Effekte ergeben.

Bevorzugte Weiterbildungen der Erfindung bzw. Varianten zu solchen Weiterbildungen sind in den abhängigen Ansprüchen beschrieben bzw. unter Schutz gestellt.

Durch eine keim/schmutzabweisende und/oder keimtötende Oberfläche der erfindungsgemäß weitergebildeten medizinischen Geräte reduziert sich die Kontaminationsgefahr bzw. die Gefahr allergischer Reaktionen, sollte ein Kontakt zwischen Geräteoberfläche und Patient entstehen.

Daneben wird die Reinigung der Geräte, insbesondere der Optik wesentlich erleichtert. Von Vorteil ist dabei auch eine antiseptisch wirkende Oberfläche bzw. Materialzusammensetzung sowie zusätzlich oder alternativ eine Oberfläche, die generell schmutzabweisend ist. Unter Schmutz sollen in diesem Zusammenhang alle Verunreinigungen der Oberfläche durch Keimbesiedelung und/oder durch aseptische Stoffe und Substanzen - wie z. B. Wasser, Fett, Eiweiß und dgl. - verstanden werden.

Eine Möglichkeit für die Herstellung und Auswahl von Materialien insbesondere bei optischen Gläsern und/oder Kunststoffen einschließlich der Schutzgläser/kunststoffe von Mikroskopen ist erfindungsgemäß das Dotieren derselben mit Metallen oder Metallsalzen, insbesondere Schwermetallsalzen, wie Silber- oder Kupfer-, Zink-, Nickel-, Mangan-, Cadmium-, Platinsalzen oder auch z.B. Salzen von Halbmetallen, wie Borsalzen oder dergleichen, die - wie an sich bekannt - insbesondere bakterizid, fungizid und virozid wirken. Die Wirkung wird dabei oft durch an die Keime abgebbare Metallionen, z.B. Silberionen erzielt.

Auch kann die Struktur der Oberfläche von Materialien, wie Gehäusen, Stativen und insbesondere von optischen Gläsern/Kunststoffen, derartig gestaltet werden, daß sie möglichst wenig Halt für allfällige Adhäsions- oder Kohäsionskräfte von septischen oder aseptischen Verunreinigungen und/oder Flüssigkeiten bietet. Eine fungostatische bzw. fungizide Ausbildung ist dabei auch für optische Bauteile im Inneren eines Mikroskops - wie an sich bekannt - z. B. für den Tropeneinsatz vorteilhaft. Eine schmutzabweisende Ausbildung ist dabei auch für optische Bauteile im Inneren eines Mikroskops zum Schutz für die optische Qualität vorteilhaft. Als Variante dazu könnten die optischen Glas/Kunststoffteile auch mit einer hauchdünnen Schicht eines transparenten Kunst- bzw. Keramikstoffes beschichtet - z. B. bedampft - sein. Dafür kommen auch Stoffe aus der Mikrosystemtechnik wie Silizium, Metallkeramiken, Metalle und Kunststoffe wie Teflon ®/Silikon o.dgl. infrage.

Zur Erhöhung der Steifigkeit und Festigkeit sind an sich bekannt: Verdichtete Keramik als Mikroskopchassis gem. EP-B1-90967. Einen Bezug auf die eingangs gestellte Aufgabe kann man daraus nicht ableiten.

Das Beschichten von Bauteilen mit bestimmten Substanzen ist grundsätzlich bekannt, wie z.B.

- das Farblackbeschichten, um ein gefälliges Aussehen zu erzielen, wie das Hammerschlaglackbeschichten zur Erhöhung der Oberflächenfestigkeit;

- das Beschichten mit Antioxidanten und Lichtschutzmitteln, z. B. in der EP-A1-745 646 beschrieben;

- das Beschichten eines Glasträgers mit synthetischen Polymeren für Bioprozesse, z.B. in der US-A-4 357 142 beschrieben;

- das Verchromen von Oberflächen zur Erhöhung der Oberflächenfestigkeit usw.

Teflonbeschichtete Objektive bzw. Objektivgehäuse sind an sich ebenso bekannt, jedoch ging es bei diesen bekannten beschichteten Objektiven um andere Aufgabenstellungen: Es handelt sich dabei um Objektive für Inversionsmikroskope, die in Petrischalen oder dgl. eingetaucht werden können und derart mit liquiden Medien in Berührung kommen. Die dort durchgeführte Teflonbeschichtung hat im Wesentlichen drei Aufgaben:

1. sie soll eine mechanische Abrasion (Kratzsicherheit) des Objektives hintanhalten;

2. sie soll dem Objektiv eine erhöhte Korrosionsfestigkeit geben;

3. sie soll verhindern, daß allfällige Metallionen des Objektivgehäuses über einen chemisch physikalischen Prozess in das liquide Medium abwandern (chemischer Schutz des Mediums gegen das eintauchende Objektiv).

Daß natürlich, abgesehen davon, die hydrophobe Eigenschaft des Teflons als willkommen in Kauf genommen wurde, ergibt sich bei der Art der liquiden Medien als selbstverständlich. Die Technik, bei der derartige Inversionsmikroskope zum Einsatz gelangen, wird unter anderem "Patch-Clamp-Technik" genannt.

Eine Beschichtung zu den eingangs angegebenen Zwecken bei medizinischen Geräten ist jedoch noch nie nahegelegt worden.

Beim Anwenden von Keramik im Sinne der Erfindung wird beispielsweise an Sinterkeramik und/oder Metallkeramik gedacht, deren Oberfläche so gestaltet ist, daß sie keim- und/oder schmutzabweisend ist. Der Fachmann hat dabei bestimmte geometrische Abmessungen zu berücksichtigen, die für den Angriff von septischen oder aseptischen Verunreinigungen günstigere oder ungünstigere Eigenschaften aufweisen. Wenngleich jede Mikrostruktur die Oberfläche eine gewisse Struktur aufweisen muß, wird sie für den Beobachter als eine "glatte Oberfläche" erscheinen, an der nichts haftet. Metallkeramiken, $Al_2O_3$ und AIN eignen sich vor allem für eine erfindungsgemäße Bedampfung.

Der Fachmann kann dabei aus einer Fülle an sich bekannter keramischer Materialien wählen, wobei dafür grundsätzlich auch moderne Materialien wie Glasmetalle mit einer Legierung, die Nickel, Zirkon und Titan enthalten. Die Atome solcher Glasmetalle haben kein regelmäßiges Kristallgitter ausgebildet. Sie sind wahllos angeordnet, woraus sich eine amorphe Struktur ergibt. C/SiC-Verbundkeramiken, wie sie beispielsweise für Spiegelstrukturen angewendet werden, können ebenso wie Oxidkeramiken zum Einsatz gelangen.

Bei einer erfindungsgemäßen speziellen Oberflächengestaltung - vor allem auch für keramische Oberflächen - wird von folgendem Prinzip ausgegangen: Die Oberfläche wird physikalisch (z.B. chemisch oder/und mikromechanisch) so strukturiert, daß unerwünschte Substanzen nicht anhaften können. Eine mögliche Ausgestaltung sieht dabei die gezielte Anordnung von Erhebungen mit bestimmten geometrischen Abmessungen vor, die Substanzen (z.B. Flüssigkeiten) aufgrund deren Oberflächenspannung keinen Halt an der Oberfläche bieten. Die Substanzen können daher an der Oberfläche keine Adhäsionskräfte freisetzen.

Vergleichbare Effekte sind in der Natur bekannt, z.B. bei der natürlichen Oberflächengestaltung von Lotusblättern (Oberflächenstruktur) oder an Silbermantelblättern (Feinstbehaarung), an denen unter Mitnahme jeglicher anliegender Schmutzpartikeln Wasser abperlt, ohne zu benetzen.

Abgesehen von der Verhinderung des Anhaftens sind solche Oberflächen zudem besonders leicht zu reinigen, da bedingt durch die Adhäsionskräfte von Reinigungsflüssigkeiten - die eben nicht mit der Oberfläche selbst reagieren können - an der Oberfläche zufällig anliegende Schmutzpartikeln an die Tröpfchen der Reinigungsflüssigkeit gebunden und damit abgespült werden.

Als ein Beispiel einer solchen Oberflächengestaltung wird eine Oberfläche mit folgenden geometrischer Struktur angegeben (wobei diese gegebenenfalls nicht notwendigerweise in mathematisch exakter Ausgestaltung vorliegen muß):

$$Sin(x) \times Cos\,(y).$$

Die sich daraus ergebende Oberfläche weist eine regelmäßige Hügelstruktur auf, deren Gipfel bevorzugt folgende Abstände aufweisen: 1-100 μm, insbesondere 3-60 μm.

Alternativ können die Erhebungen auch einen kubischen oder einen kegelförmigen Aufbau aufweisen.

Die Erfindung umfaßt jedoch auch Mischformen, die der Fachmann in Routineversuchen ermitteln kann.

Die Wirksamkeit solcher erfindungsgemäß geometrisch ausgebildeter Flächen kann durch die Kombination mit anderen erfindungsgemäßen Maßnahmen, wie keimtötende Ausrüstung (z.B. Metall- oder Metallsalzbeschichtung) verstärkt werden.

Eine besondere Ausgestaltung ergibt sich dabei, wenn die mikromechanischen Erhebungen aus unterschiedlichen Elementen (insbes. Metallen oder Halbmetallen) aufgebaut sind, die gegebenenfalls voneinander elektrisch isoliert sind, so daß sich derart zu den mechanischen und zu den an sich bioziden Eigenschaften der entsprechenden Metalle noch elektrische bzw. elektrolytische Wirkungen einstellen, die für bestimmte Keime wiederum besonders toxisch bzw. abweisend wirken.

Eine andere Möglichkeit, herkömmliche Geräte, insbesondere Stative erfindungsgemäß auszurüsten ist die Beschichtung derselben mit der Matrix eines Trägerstoffes, in den keimtötende oder keimabweisende Substanzen eingebracht sind. Unter keimtötenden Substanzen werden all jene Substanzen verstanden, die herkömmlich oder zukünftig zum Keimtöten bzw. zum Desinfizieren eingesetzt werden. Dies können die oben erwähnten Metallsalzverbindungen, aber auch Alkohole, oxidierende Stoffe, Zellmembran schädigende Polyelektrolyte - z.B. Tenside - o.dgl. oder Mischungen daraus sein.

Diese erfindungsgemäße Ausgestaltung steht gewissermaßen in konträrem Gegensatz zu den Beschichtungen gemäß der oben erwähnten EP-A1-745 646.

Unter Trägerstoffen sind all jene Stoffe zu verstehen, die geeignet sind, andere Stoffe, nämlich keimtötende Substanzen, aufzunehmen. Es kann sich dabei um Schäume oder Gewebe oder sonstige Strukturen handeln, in die die gesagten Substanzen eingebracht bzw. ein- und/oder angebunden werden können. Im weitesten Sinne kann die Matrix des Trägerstoffes auch so aufgebaut sein, daß sie an ihrer Oberfläche keimtötende oder - abweisende Substanzen oder dergleichen hält. Demzufolge können solche Trägerstoffe aus Kunststoff, Gummi, Lacken, Keramik oder dergleichen aufgebaut sein. Beispielsweise kann es sich auch um Antifoulinglacke handeln, die heute schon z. B. im Bootsbau gewisse keim- und pflanzenbewuchsabweisende Eigenschaften aufweisen.

Eine weitere, an und für sich unabhängige Idee, die jedoch allein oder insbesondere in Kombination mit den vorerwähnten erfindungsgemäßen Ideen sinnvoll anwendbar ist , ist die Oberfläche oder Teile - z.B. darunterliegende - davon elektrisch leitfähig auszubilden. Dies zum einen, weil dadurch die Oberfläche antistatisch ausgebildet sein kann und zum anderen die Oberfläche über das Widerstandsprinzip auch künstlich auf eine keimtötende Temperatur gebracht werden kann, die zumindest wärmelabile Keime tötet. Bei der elektrostatischen Ausrüstung einer erfindungsgemäßen Oberfläche sind verschiedene Faktoren insbesondere die Spannungsverhältnisse bzw. elektrostatischen Ladungsverhältnisse in der Umgebung zu berücksichtigen. Zur Erzielung elektrischer bzw. elektrothermischer oder elektrostatischer Eigenschaften kann in die Oberfläche leitfähiges Material wie z.B. Kohlenstoff eingebunden werden. Gegebenenfalls kann dabei auch hinsichtlich ihrer Antitoxizität Aktivkohle mit großer innerer Oberfläche herangezogen werden.

Das anti-elektrostatische Beschichten von Mikroskopen ist durch die Anmelderin z.B. in der WO-A1-95/19583 bekannt gemacht worden. Verschiedene Mikroskope, wie LEICA MS5, MZ6 und MZ8 sind in "ESD-Ausführung" (Electro Static Discharge) auf dem Markt. Allerdings wird durch diese bekannte antistatische Beschichtung die erfindungsgemäße Aufgabe nicht gelöst, wenn gleich - zufällig - die zum Schutz von elektronischen Bauteilen vorgesehene antistatische Beschichtung auch zu einer Reduktion von Staub- und Schmutzablagerungen an den Geräten führt. Die Kontaktsterilität wird dadurch jedoch grundsätzlich noch nicht verbessert.

In einem anderen Bereich ist auch schon zu einem anderen Zweck von der Anmelderin ein Retroreflektor mit einer leitenden Beschichtung ausgerüstet worden, um diesen oberflächlich aufzuheizen, um dadurch Eisbildung und Dampfniederschlag zu verhindern. Vgl. WO-A1-96/33428. Eine Keimtötende Wirkung war dazu jedoch noch nicht vorgesehen.

Bei Anwendung der erfindungsgemäßen leitenden Widerstandsbeschichtung muß auf die Temperaturfestigkeit des Trägermaterials geachtet werden. Die gezielte Anwendung von z. B. Polymeren mit wärmeformbeständigkeitserhöhenden Füllstoffen wie z.B. Glimmer wird diesbezüglich angeregt. Ein zusammenfassender Artikel über verschiedenste Füllstoffe ist angegeben in "Kunststoffe 87 (1997) 9, Carl Hanser Verlag, München Seite 1106-1112", worauf ausdrücklich bezug genommen wird.

Die Erfindung ist nicht auf die Anwendung der einzelnen erfinderischen Elemente eingeschränkt, sondern auch eine Kombination derselben kann erfindungsgemäße symbiotische, antiseptische bzw. keim/schmutzabweisende Effekte ergeben.

Unter Optik im Sinne der Erfindung sind alle optischen Bauteile wie Gläser, Kunststoffe und auch Spiegel, die auch aus Metall sein können, gemeint.

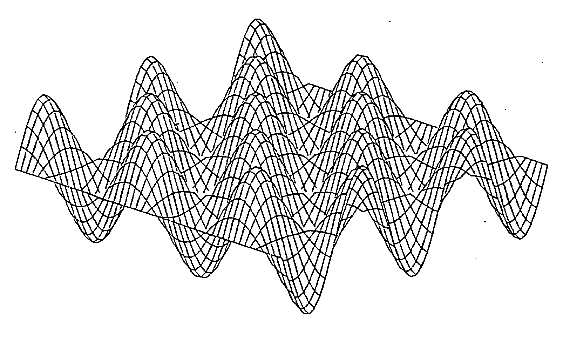

Ein Beispiel einer erfindungsgemäßen Oberfläche ist in der Zeichnung dargestellt.

Es handelt sich dabei beispielhaft um eine sin/cos-förmige Oberfläche mit gleichartigen Erhebungen und Vertiefungen. In Abhängigkeit von den Abmessungen zwischen den Erhebungen ist eine derart ausgebildete Oberfläche geeignet, Adhäsionskräfte abzuweisen bzw. rein mechanisch das Anlegen und Verklumpen von Keimen zu verhindern. Im Rahmen der Erfindung könnten jedoch auch auf die nach unten ragenden Täler verzichtet werden.

Zur besseren optischen Darstellung sind die Erhebungen und Täler mit Gitternetzlinien gezeichnet.

Im Rahmen der Erfindung liegen auch besondere Ausgestaltungen, bei denen die Erhebungen bzw. Täler aus mehreren unterschiedlichen Materialien aufgebaut sind, derart, daß benachbarte Erhebungen unterschiedlich sind, oder daß Teile an Erhebungen unterschiedlich sind. So sind z.B. entlang bestimmter Gitternetzlinien oder entlang von nicht dargestellten Höhenlinien Grenzen verschiedener Materialien, insbes verschiedener Metalle denkbar.

Wenn die Metalle voneinander isoliert sind, ergeben sich elektrolytische Vorgänge bei berührenden, Ionenleitfähigen Substanzen, die wiederum antiseptisch wirken können.

**Patentansprüche**

1. Operationsmikroskop und/oder Stativ dazu, **dadurch**

   **gekennzeichnet, dass** die Oberfläche mit einer Vielzahl von Erhebungen bzw. Vertiefungen versehen ist, deren Gipfel bzw. Täler zueinander einen Abstand von 1-100 $\mu$m, bevorzugt von 3-60 $\mu$m aufweisen, wobei der Abstand innerhalb des angegebenen Bereichs variieren kann, und dass entweder die Erhebungen bzw. Täler eine kubische mit geraden Kanten oder eine zylindrische, kegelige oder kegelstumpfartige Struktur mit zylindrischen oder kegelmantelartigen Flächen umfassen, oder die Erhebungen bzw. Täler der Oberfläche nach sin/cos-förmigen Kurven gestaltet sind, insbesondere nach der Formel: sin(x) x cos(y), wobei x = 1-100 $\mu$m oder .3-60 $\mu$m und y = 1-100 $\mu$m oder 3-60 $\mu$m.

2. Operationsmikroskop nach Anspruch 1, **dadurch gekennzeichnet, dass** die Erhebungen aus unterschiedlichen Materialien, insbesondere aus unterschiedlichen Metallen, Halbmetallen oder Metallsalzen aufgebaut sind.

3. Operationsmikroskop nach Anspruch 2 **dadurch gekennzeichnet, dass** die Metalle, Halbmetalle oder Metallsalze voneinander elektrisch isoliert sind.

4. Operationsmikroskop nach Anspruch 2, **dadurch gekennzeichnet, dass** die Erhebungen unterschiedliche Schichten von Metallen, Halbmetallen oder Metallsalzen aufweisen.

5. Operationsmikroskop nach einem der vorhergehenden Ansprüche, **dadurch gekennzeichnet, dass** das Material des Operationsmikroskops und/oder die Oberfläche keimtötend ausgebildet bzw. beschichtet ist.

6. Operationsmikroskop nach einem der vorhergehenden Ansprüche, **dadurch gekennzeichnet, dass** seine Oberfläche mindestens ein antiseptisches Agens enthält oder damit beschichtet ist.

7. Operationsmikroskop nach einem der vorhergehenden Ansprüche, **dadurch gekennzeichnet, dass** seine Oberfläche, insbesondere das Material und/oder die Oberfläche optischer Gläser oder Kunststoffe mit antiseptischen Substanzen dotiert ist.

8. Operationsmikroskop nach Anspruch 7, **dadurch gekennzeichnet, dass** seine Oberfläche, insbesondere das Material und/oder die Oberfläche optischer Gläser oder Kunststoffe mit Salzen von Schwermetallen und/oder Halbmetallen dotiert ist.

9. Operationsmikroskop nach Anspruch 8, **dadurch gekennzeichnet, dass** seine Oberfläche, insbesondere das Material und/oder die Oberfläche optischer Gläser oder Kunststoffe mit Silber- oder Borsalzen dotiert ist.

**Claims**

1. Operation microscope and/or limb thereto, **characterized in that** the surface is provided with a multiplicity of elevations and depressions, the peaks and troughs of which having a distance from one another of 1-100 $\mu$m, preferably 3-60 $\mu$m, with it being possible for the distance to vary within the specified range, and **in that** either the elevations and the troughs comprise a cubic structure with straight edges or a cylindrical, conical or frustum-like structure with cylindrical or cone-shaped, shell-like areas or the elevations and the troughs of the surface are shaped in accordance with sinusoidal curves, in particular in accordance with the formula sin(x)cos(y), with x = 1-100 $\mu$m or 3-60 $\mu$m and y = 1-100 $\mu$m or 3-60 $\mu$m.

2. Operation microscope according to Claim 1, **characterized in that** the elevations are constructed from different materials, in particular from different metals, metalloids or metal salts.

3. Operation microscope according to Claim 2, **characterized in that** the metals, metalloids or metal salts are electrically isolated from one another.

4. Operation microscope according to Claim 2, **characterized in that** the elevation have different layers of metals, metalloids or metal salts.

5. Operation microscope according to one of the preceding claims, **characterized in that** the material of the operation microscope and/or the surface is germicidal or coated to that effect.

6. Operation microscope according to one of the preceding claims, **characterized in that** its surface contains at least one antiseptic agent or is coated therewith.

7. Operation microscope according to one of the preceding claims, **characterized in that** its surface, in particular the material and/or the surface of optical glass or plastics, is doped with antiseptic substances.

8. Operation microscope according to Claim 7, **characterized in that** its surface, in particular the material and/or the surface of optical glass or plastics, is doped with salts of heavy metals and/or metalloids.

9. Operation microscope according to Claim 8, **characterized in that** its surface, in particular the material and/or the surface of optical glass or plastics, is doped with silver or boron salts.

**Revendications**

1. Microscope d'opération et/ou support pour celui-ci, **caractérisé en ce que** la surface est pourvue d'une pluralité de surélévations ou de creux, dont les sommets ou les vallées présentent entre eux/elles une distance de 1 à 100 $\mu$m, de préférence de 3 à 60 $\mu$m, la distance pouvant varier à l'intérieur de la zone indiquée, et **en ce que** soit les surélévations soit les vallées comprennent une structure cubique avec des arêtes droits ou une structure cylindrique, conique ou tronconique avec des faces cylindriques ou tronconiques, ou les surélévations ou les vallées de la surface sont configurées selon des courbes sinusoïdales, en particulier selon la formule sin(x)cos(y), dans laquelle x = 1 a 100 $\mu$m ou 3 à 60 $\mu$m et y = 1 à 100 $\mu$m ou 3 à 60 $\mu$m.

2. Microscope d'opération selon la revendication 1, **caractérisé en ce que** les surélévations sont réalisées en matériaux différents, en particulier en métaux, en métalloïdes ou en sels métalliques différentes.

3. Microscope d'opération selon la revendication 2, **caractérisé en ce que** les métaux, métalloïdes ou sels métalliques sont électriquement isolés les uns des autres.

4. Microscope d'opération selon la revendication 2, **caractérisé en ce que** les surélévations présentent différentes couches de métaux, de métalloïdes ou de sels métalliques.

5. Microscope d'opération selon l'une quelconque des revendications précédentes, **caractérisé en ce que** la matière du microscope d'opération et/ou à surface est constituée ou revêtue de matière germicide.

6. Microscope d'opération selon l'une quelconque des revendications précédentes, **caractérisé en ce que** sa surface, contient au moins un agent antiseptique ou est revêtue de celui-ci.

7. Microscope d'opération selon l'une quelconque des revendication précédentes, **caractérisé en ce que** sa surface, en particulier la matière et/ou la surface des verres ou des matières plastiques optiques, est dopée avec des substances antiseptiques.

8. Microscope d'opération selon la revendication 7, **caractérisé en ce que** sa surface, en particulier la matière et/ou la surface, des verres ou des matières plastiques optiques, est dopée avec des sels de métaux lourds et/ou de métalloïdes.

9. Microscope d'opération selon la revendication 8, **caractérisé en ce que** sa surface, en particulier la matière et/ou la surface des verres ou des matières plastiques optique, est dopée avec des sels d'argent ou de bore.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 4413920 A1
- WO 9211043 A
- US 5681575 A
- EP 90967 B1
- EP 745646 A1
- US 4357142 A
- WO 9519583 A1
- WO 9633428 A1

### In der Beschreibung aufgeführte Nicht-Patentliteratur

- Kunststoffe. Carl Hanser Verlag, 1997, vol. 87, 1106-1112